# EUROPEAN PATENT SPECIFICATION

(11) **EP 4 291 170 B1**
(45) Date of publication and mention of the grant of the patent: **13.05.2026**
(21) Application number: 22703438.6
(22) Date of filing: 27.01.2022
(51) Int. Cl.: A61K 31/05, A61P 25/08

(54) **USE OF CANNABIDIVARIN IN THE TREATMENT OF SEIZURES ASSOCIATED WITH CANINE EPILEPSY**
VERWENDUNG VON CANNABIDIVARIN BEI DER BEHANDLUNG VON ANFÄLLEN IM ZUSAMMENHANG MIT EPILEPSIE BEI HUNDEN
UTILISATION DE LA CANNABIDIVARINE DANS LE TRAITEMENT DES CRISES ASSOCIÉES À L'ÉPILEPSIE CANINE

(30) Priority: 12.02.2021 GB 202102010
(43) Date of publication of application: 20.12.2023
(73) Proprietor: Jazz Pharmaceuticals Research UK Limited, Sittingbourne, Kent ME9 8AG (GB)
(72) Inventor: TSE, Karen Ka-Yen, Sittingbourne, Kent ME9 8AG (GB)
(74) Representative: HGF
(86) International application number: PCT/GB2022/050221
(87) International publication number: WO 2022/171981

(56) References cited:
- WO-A1-2015/198078
- GB-A- 2 487 183
- MEIR BIALER ET AL: "Progress report on new antiepileptic drugs: A summary of the Twelfth Eilat Conference (EILAT XII)", EPILEPSY RESEARCH, vol. 111, 1 March 2015 (2015-03-01), NL, pages 85 - 141, XP055611172, ISSN: 0920-1211, DOI: 10.1016/j.eplepsyres.2015.01.001
- M. PODELL ET AL: "2015 ACVIM Small Animal Consensus Statement on Seizure Management in Dogs", JOURNAL OF VETERINARY INTERNAL MEDICINE, vol. 30, no. 2, 22 February 2016 (2016-02-22), US, pages 477 - 490, XP055599173, ISSN: 0891-6640, DOI: 10.1111/jvim.13841

## Description

### FIELD OF THE INVENTION

The present invention relates to the use of cannabidivarin (CBDV) for the treatment of seizures associated with canine epilepsy.

The CBDV used is in the form of a highly purified extract of cannabis such that the CBDV is present at greater than 95% of the total extract (w/w) and the cannabinoid tetrahydrocannabinol (THC) has been substantially removed, to a level of not more than 1.5% (w/w).

Preferably the CBDV used is in the form of a botanically derived purified CBDV which comprises greater than or equal to 95% (w/w) CBDV and less than or equal to 5% (w/w) other cannabinoids, wherein the less than or equal to 5% (w/w) other cannabinoids comprise the cannabinoids tetrahydrocannabinol (THC); tetrahydrocannabivarin (THCV); cannabidiol-C1 (CBD-C1); cannabidiol (CBD); cannabidivarin acid (CBDVA) and cannabidiol-C4 (CBD-C4). Alternatively, a synthetically produced CBDV is used.

### BACKGROUND TO THE INVENTION

Epilepsy is reported to be the most common neurological disorder in dogs and has been estimated to affect approximately 0.75% of the canine population.¹

A limited number of existing anti-epileptic drugs (AEDs) have been approved for the treatment of epilepsy in dogs worldwide.² The 2015 American College of Veterinary Internal Medicine consensus statement on seizure management in dogs indicates that anticonvulsant treatment should be initiated with either one of the AEDs phenobarbital or potassium bromide. However, a 2008 study found that treatment of canine epilepsy by a combination of the two AEDs is ineffective in reducing seizures in about 20% to 30% of dogs.³ Increasing dosage may improve seizure control but due to side effects and toxicity (polyuria/polydipsia, polyphagia, ataxia, lethargy and hepatoxicity), this is not possible. The ineffectiveness and adverse effects of these drugs have led to the search for more effective treatments.

A number of different underlying diseases and factors can cause seizures leading to epilepsy in dogs. The condition can be inherited (genetic or idiopathic epilepsy), caused by structural problems in the brain (structural epilepsy), or stem from an unknown cause (epilepsy of unknown cause).

The main symptom of epilepsy is repeated seizures. In order to determine the type of seizures, clinical observations and electroencephalography (EEG) tests are conducted and the type(s) of seizures in humans can be classified according to the International League Against Epilepsy (ILEA) classification.

Although such classification systems exist for human seizures, there is not yet a widely accepted classification system available for seizures in dogs. Recently, the International Veterinary Epilepsy Task Force proposed a classification scheme for veterinary seizures,⁴ similar to the current human ILAE classification system.

Focal epileptic seizures originate in a discrete area of the brain and are characterized by signs that affect a single side or specific part of the body. Focal epileptic seizures can present in the following ways in dogs: i) motor (episodic focal motor phenomena e.g. facial twitches, repeated jerking head movements, rhythmic blinking, twitching of facial musculature or repeated rhythmic jerks of one extremity); ii) autonomic (parasympathetic and epigastric components e.g. dilated pupils, hypersalivation or vomiting); and iii) behavioural (short lasting episodic change in behaviour e.g. anxiousness, restlessnesss, unexplainable fear reactions or abnormal attention seeking/'clinging' to the owner.)

Generalised epileptic seizures are characterised by bilateral involvement of both sides of the body as both cerebral hemispheres are involved. In dogs and cats, generalized epileptic seizures predominantly present as tonic, clonic or tonic-clonic epileptic seizures with possible loss of consciousness. Furthermore salivation, urination and/or defecation often occur. Animals may also experience other generalised convulsive epileptic seizures such as myoclonic seizures (jerking movements usually affecting both sides of the body). Non-convulsive generalized epileptic seizures experienced by animals include atonic seizures, whereby the sudden and general loss of muscle tone causes the animal to collapse.

Focal epileptic seizures can spread from initial regional cerebral involvement to bilateral cerebral involvement (focal epileptic seizures evolving into generalised epileptic seizures). The seizure starts with regional motor, autonomic and/or behavioural signs and then is rapidly followed by a convulsive stage with bilateral tonic, clonic or tonic-clonic activity and loss of consciousness. This is the most common seizure type observed in the dog. The onset of the focal epileptic seizure is often very short (seconds to minutes) after which follows the secondary generalisation with convulsions. The focal epileptic seizure onset may be difficult to detect due to its brief nature.

Cannabidiol (CBD), a non-psychoactive derivative from the cannabis plant, has demonstrated anti-convulsant properties in pre-clinical and clinical studies both in animal models and humans. Three randomized control trials showed efficacy of the purified pharmaceutical formulation of CBD in patients with Dravet and Lennox-Gastaut syndrome.

Based on these three trials, a botanically derived purified CBD preparation was approved by the FDA in June 2018 and the EMA in September 2019 for the treatment of seizures associated with Dravet and Lennox-Gastaut syndromes. In July 2020, the FDA approved the drug for the treatment of seizures associated with Tuberous Sclerosis Complex.

A clinical trial in 2019 investigated the effect of oral CBD administration in addition to conventional antiepileptic treatment on seizure frequency in dogs with idiopathic epilepsy.⁵ CBD-infused oil was administered twice daily at 2.5 mg/kg to nine dogs in the CBD group and noninfused oil to seven dogs in the placebo group. The CBD-infused oil was derived from industrial hemp, containing 100 mg of CBD/mL along with trace amounts of the other cannabinoids. Additional ingredients included cold-pressed hemp oil and oil-miscible chicken flavouring. The plants used were said to be low- tetrahydrocannabinol (THC) plants containing less than 0.3% THC. Seizure frequencies were recorded by respective dog owners. The investigators found that the number of dogs classified as responders to the treatment (more than 50% decrease in seizure activity) was low, only 2 out of 9 dogs (22%). This was the same as the number of responders in the placebo group, 2 out of 7 dogs (29%). The proportion of responders was in fact higher in the placebo group. Thus, this study suggests a lack of efficacy when CBD was used to treat canine epilepsy. One of the limitations of such a study was the reliance on dog owners for recording seizure frequency, as acknowledged by the author, as some seizures could have been missed if they were nocturnal or the owner was absent.

Cannabidivarin (CBDV) is a homolog of CBD, with a propyl side-chain. It has previously been reported that CBDV may have anticonvulsant activity in rats and mouse. Several clinical trials are underway investigating the safety, tolerability and efficacy in humans.

A study by Hill *et al.* in 2013 investigated the anticonvulsant profiles of cannabis-derived botanical drug substances (BDSs) rich in CBDV and containing CBD in mouse and rat seizure models.⁶ They found that such extracts are anticonvulsant in mouse and rat and act via a CB1 receptor-independent mechanism. CBDV BDS contained 57.8% CBDV and 13.7% CBD and remaining content comprised plant matter. A previous study by Hill *et al.* in 2012⁷ looked at the effects of CBDV in rodent seizure models but was not found to have any effect in pilocarpine-induced seizures.

Berk *et al.* (2018)⁸ investigated the use of dietary supplements in dogs with idiopathic epilepsy (IE) whereby a web-based owner questionnaire assessed how and why owners of dogs with IE use different dietary regimes. Cannabis/hemp oil is mentioned among a list of different oils used by owners but with no mention of the composition of the oil used. The author further discusses how cannabis can be toxic for dogs and additional studies on CBD's efficacy and safety are required.

WO2011/121351 discloses the use of isolated CBDV in *in vitro* epileptiform models in rat hippocampal brain slices as well as in *in vivo* models in rats using pentylenetetrazole (PTZ)-induced and pilocarpine-induced seizure models. The document discloses use of CBDV alone and in combination with other AEDs.

Another study looked at CBDV effect in postnatal day 10 and 20 rats and acute neurotoxicity in immature rats.⁹ They found that synthetic CBDV displayed an age and model-specific profile of anticonvulsant action.

In 2018, a Phase II study evaluated the safety and efficacy of CBDV to treat focal seizures in humans.¹⁰ It was found that the CBDV and placebo groups showed similar reductions in focal seizures of approximately 40%. Thus, there was no difference between placebo and treatment groups in this patient population.

The applicant has found that treatment with highly purified CBDV resulted in a significant reduction in seizures in canine epilepsy. This is surprising as this is the first time it has been demonstrated that this cannabinoid may be of therapeutic value to treat canine epilepsy.

Meir Bialer et al.: "Progress report on new antiepileptic drugs: A summary of the Twelfth Eilat Conference (EILAT XII)", EPILEPSY RESEARCH, vol. 111, 1 March 2015 (2015-03-01), pages 85-141, discusses CBDV having an anti-convulsant / anti-seizure activity in animals and further discusses the results of toxicology tests of CBDV in rats and dogs.

M. Podell et al.: "2015 ACVIM Small Animal Consensus Statement on Seizure Management in Dogs", JOURNAL OF VETERINARY INTERNAL MEDICINE, vol. 30, no. 2, 22 February 2016 (2016-02-22), pages 477-490, discusses the treatment seizures in dogs.

WO2015/198078A1 discusses the use of 7-OH-CBDV for use in the treatment of epilepsy.

GB2487183A discusses the use of the phytocannabinoid cannabidivarin (CBDV) and combinations of the phytocannabinoid CBDV with tetrahydrocannabivarin (THCV) and cannabidiol (CBD) in the treatment of epilepsy.

### BRIEF SUMMARY OF THE DISCLOSURE

The invention is defined by the claims. Any subject matter falling outside the scope of the claims is provided for information purposes only. Any references in the description to methods for treatment of the human or animal body by surgery or therapy refer to the compounds and pharmaceutical compositions of the present invention for use in the methods for treatment of the human or animal body by surgery or therapy.

In accordance with a first aspect of the present invention there is provided a cannabidivarin (CBDV) preparation for use in the treatment of seizures associated with canine epilepsy, wherein the dose of CBDV is between 75 mg/kg/day and 125 mg/kg/day, wherein the CBDV preparation comprises greater than 95% (w/w) CBDV and not more than 1.5% (w/w) tetrahydrocannabinol (THC).

In a further embodiment the seizures associated with canine epilepsy are tonic, clonic, tonic-clonic, atonic, myoclonic, absence, focal seizures without impairment, focal seizures with impairment, and focal seizures with secondary generalisation.

Preferably the CBDV preparation comprises greater than or equal to 95% (w/w) CBDV and less than or equal to 5% (w/w) other cannabinoids, wherein the less than or equal to 5% (w/w) other cannabinoids comprise the cannabinoids tetrahydrocannabinol (THC); tetrahydrocannabivarin (THCV); cannabidiol-C1 (CBD-C1); cannabidiol (CBD); cannabidivarin acid (CBDVA) and cannabidiol-C4 (CBD-C4).

Preferably the CBDV preparation is used in combination with one or more concomitant anti-epileptic drugs (AED).

In one embodiment the CBDV is present is isolated from cannabis plant material. Preferably at least a portion of at least one of the cannabinoids present in the CBDV preparation is isolated from cannabis plant material.

In a further embodiment the CBDV is present as a synthetic preparation. Preferably at least a portion of at least one of the cannabinoids present in the CBDV preparation is prepared synthetically.

The dose of CBDV is between 75 mg/kg/day and 125 mg/kg/day.

Preferably the dose of CBDV is 100 mg/kg/day.

Also disclosed, but not claimed, is a method of treating seizures associated with canine epilepsy comprising administering a cannabidivarin (CBDV) preparation to the subject in need thereof.

### BRIEF DESCRIPTION OF THE DRAWINGS

Embodiments of the invention are further described hereinafter with reference to the accompanying drawings, in which:
Figures 1 to 2 show quantified EEG in the dog.
Figure 1.1 shows the effects of diazepam on cortex at 0-10 min; Figure 1.2 at 10-20 min; Figure 1.3 at 20-30 min; Figure 1.4 at 30-40 min; Figure 1.5 at mean 10-min periods.
Figure 2.1 shows the effects of CBDV on cortex at 0-10 min; Figure 2.2 at 10-20 min; Figure 2.3 at 20-30 min; Figure 2.4 at 30-40 min; Figure 2.5 at mean 10-min periods.

### DEFINITIONS

Definitions of some of the terms used to describe the invention are detailed below:

Over 100 different cannabinoids have been identified, see for example, Handbook of Cannabis, Roger Pertwee, Chapter 1, pages 3 to 15. These cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoids" are defined as cannabinoids that have been extracted from the cannabis plant and purified to the extent that other cannabinoids and non-cannabinoid components that are co-extracted with the cannabinoids have been removed, such that the highly purified cannabinoid is greater than or equal to 95% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

"Treatment-resistant epilepsy" (TRE) or "intractable epilepsy" is defined as per the ILAE guidance of 2009 as epilepsy that is not adequately controlled by trials of one or more AED.

"Tonic seizures" can be generalised onset, affecting both sides of the brain, or they can be focal onset, starting in just one side of the brain. If a tonic seizure starts in both sides of the brain, all muscles tighten and the subject's body goes stiff. If standing, they may fall to the floor, their neck may extend, eyes open wide and roll upwards, whilst their arms may raise upwards and legs stretch or contract. If a tonic seizure starts in one side of the brain muscles tighten in just one area of the body. Tonic seizures usually last less than one minute.

"Clonic seizures" are characterised by sustained rhythmical jerking. During a clonic seizure, jerking of the body or parts of the body are the main symptom. They can begin in one area or affect both sides of the brain. Whilst such seizures are rare, they cannot be stopped by restraining the person.

"Tonic-clonic seizures" consist of two phases: the tonic phase and the clonic phase. In the tonic phase the body becomes entire rigid, and in the clonic phase there is uncontrolled jerking. Tonic-clonic seizures may or may not be preceded by an aura, and are often followed by headache, confusion, and sleep. They may last mere seconds or continue for several minutes. These seizures are also known as a grand mal seizure.

"Atonic seizures" occur when a person suddenly loses muscle tone so their head or body may go limp. They are also known as drop attacks. In some children, only their head drops suddenly. They can begin in one area or side of the brain (focal onset) or both sides of the brain (generalized onset).

"Myoclonic seizures" are characterised by a 'muscle jerk'. Myoclonic seizures are brief but can happen in clusters (many happening close together in time) and often happen shortly after waking. In myoclonic seizures the person is conscious, but they are classified as generalised seizures.

"Absence seizures" also may be called "petit mal" seizures. These types of seizure cause a loss of awareness for a short time. They mainly affect children although can happen at any age. During an absence seizure, a person may: stare blankly into space; look like they're "daydreaming"; flutter their eyes; make slight jerking movements of their body or limbs. The seizures usually only last up to 15 seconds and may occur several times a day.

"Focal Seizures" are defined as seizures which originate within networks limited to only one hemisphere. What happens during the seizure depends on where in the brain the seizure happens and what that part of the brain normally does.

"Focal seizures without impairment" are seizures which originate within networks limited to only one hemisphere where the awareness or responsiveness of the subject is not impaired.

"Focal seizure with impairment" usually start in a small area of the temporal lobe or frontal lobe of the brain and involve other areas of the brain within the same hemisphere that affect alertness and awareness. Most subjects experience automatisms during a focal seizure with impaired consciousness.

"Focal seizure with secondary generalisation" start in a limited area on one side of the brain and spread to involve both sides. This is different from a generalized onset seizure, which starts on both sides of the brain.

"Epileptic spasm", "spasms", "infantile spasm", "juvenile spasm" or "West syndrome" is defined as sudden flexion, extension or mixed flexion-extension of proximal and truncal muscles, lasting 1-2 seconds. Spasms typically occur in a series, usually on wakening. Subtle forms may occur with only chin movement, grimacing, or head nodding. Spasms may be bilaterally symmetric, asymmetric, or unilateral, depending on whether they are generalised onset or focal onset.

"Generalized Seizures" affect both sides of the brain or groups of cells on both sides of the brain at the same time. This term includes seizures types like tonic-clonic, absence, or atonic.

"Unknown onset seizures" occur when the beginning of the seizure is not known. A seizure could also be called an unknown onset if it is not witnessed or seen by anyone, for example when seizures happen at night or in a person who lives alone.

Seizures can also be described by whether motor symptoms occur i.e. whether movements happen during a seizure, known as a "motor seizure." When no motor symptoms happen, it can be called a "non-motor seizure."

For focal onset seizures, motor seizures may include clonic, atonic, tonic, myoclonic seizures, or epileptic spasms. There may also be automatisms or repeated automatic movements, like clapping or rubbing of hands, lip-smacking or chewing, or running. Non-motor symptoms may include changes in sensation, emotions, thinking or cognition, autonomic functions, or lack of movement (behavior arrest).

For generalized onset seizures, motor seizures usually consist of tonic-clonic seizures. Other types also include clonic, atonic, tonic, myoclonic seizures or epileptic spasms. Non-motor symptoms are usually absence seizures. Absence seizures can also have myoclonus that can affect a specific part of the body or just the eyelids.

For unknown onset seizures, motor seizures are described as either tonic-clonic or epileptic spasms. Non-motor seizures usually include a behavior arrest.

### DETAILED DESCRIPTION

### PREPARATION OF HIGHLY PURIFIED CBDV EXTRACT

### Overview of the process

The following describes the production of the botanically derived purified CBDV (>95% w/w) which has a known and constant composition which was used in the Example below.

Plant material harvested from the *Cannabis sativa* L. plant was subjected to liquid carbon dioxide extraction, to produce a botanical extract containing CBDV in addition to other cannabinoids and non-cannabinoid components. The extract was then further purified by a solvent crystallization method to yield botanically derived purified CBDV. The crystallization process specifically removed other cannabinoids and plant components to yield greater than 95% (w/w) CBDV.

Both the botanical starting material and the botanical extract may be controlled by specifications.

An exemplary CBDV preparation of botanically derived purified CBDV is described in Table 1.1 below. In some embodiments, the isomeric content for each cannabinoid may also be specified.

**Table 1.1: Specification of an exemplary botanically derived purified CBDV preparation**

| **Test** | **Test Method** | **Limits** |
|---|---|---|
| Appearance | Visual | Off-white / pale yellow crystals |
| Identification A | HPLC-UV | Retention time of major peak corresponds to certified CBDV Reference Standard |
| Identification B | GC-FID/MS | Retention time and mass spectrum of major peak corresponds to certified CBDV Reference Standard |
| Identification C | FT-IR | Conforms to reference spectrum for certified CBDV Reference Standard |
| Identification D | Melting Point | 115 - 118°C |
| Identification E | Specific Optical Rotation | Conforms with certified CBDV Reference Standard; -110° to -140° (in 95% ethanol) |
| Total Purity | Calculation | ≥ 95.0% |
| Chromatographic Purity 1 | HPLC-UV | ≥ 95.0% |
| Chromatographic Purity 2 | GC-FID/MS | ≥ 95.0 % |
| CBD | HPLC-UV | NMT 4.0% w/w |
| CBD-C4 | | NMT 0.2% w/w |
| CBD-C1 | | NMT 0.15% w/w |
| CBDVA | | NMT 0.15% w/w |
| Δ⁹ THC | | NMT 0.05% w/w |
| THCV | | NMT 0.01% w/w |
| Residual Solvents: | GC | |
| Alkane - Ethanol | | NMT 0.5% w/w |
| | | NMT 0.5% w/w |
| Residual Water | Karl Fischer | NMT 1.0% w/w |

| | | |
|---|---|---|
| i.NMT- Not more than | | |

The purity of the botanically derived purified CBDV preparation was greater than or equal to 95%. The botanically derived purified CBDV includes THC and other cannabinoids, e.g., CBD, CBDVA, THCV, CBD-C1, and CBD-C4.

Distinct chemotypes of the *Cannabis sativa* L. plant have been produced to maximize the output of the specific chemical constituents, the cannabinoids. Certain chemovars produce predominantly CBDV. Only the (-)-trans isomer of CBDV is believed to occur naturally. During purification, the stereochemistry of CBDV is not affected.

### Production of CBDV botanical drug substance

An overview of the steps to produce a botanical extract, the intermediate, are as follows:

| | |
|---|---|
| a. | Growing |
| b. | Direct drying |
| c. | Decarboxylation |
| d. | Extraction - using liquid CO₂ |
| e. | Winterization using ethanol |
| f. | Filtration |
| g. | Evaporation |

High CBDV chemovars were grown, harvested, dried, baled and stored in a dry room until required. The botanical raw material (BRM) was finely chopped using an Apex mill fitted with a 1 mm screen. The milled BRM was stored in a freezer prior to extraction.

Decarboxylation of CBDVA to CBDV was carried out using heat. BRM was decarboxylated at 115°C for 60 minutes.

Extraction was performed using liquid CO₂ to produce botanical drug substance (BDS), which was then crystalized to produce the test material. The crude CBDV BDS was winterized to refine the extract under standard conditions (2 volumes of ethanol at -20°C for approximately 50 hours). The precipitated waxes were removed by filtration and the solvent was removed to yield the BDS.

### Production of botanically derived purified CBDV preparation

The manufacturing steps to produce the botanically derived purified CBDV preparation from BDS were as follows:
a. Crystallization using C₅-C₁₂ straight chain or branched alkane
b. Filtration
c. Vacuum drying

The BDS produced using the methodology above was dispersed in C₅-C₁₂ straight chain or branched alkane. The mixture was manually agitated to break up any lumps and the sealed container then placed in a freezer for approximately 48 hours. The crystals were isolated via vacuum filtration, washed with aliquots of cold C₅-C₁₂ straight chain or branched alkane, and dried under a vacuum of <10mb at a temperature of 60°C until dry. The botanically derived purified CBDV preparation was stored in a freezer at -20°C in a pharmaceutical grade stainless steel container, with FDA food grade approved silicone seal and clamps.

Clearly a CBDV preparation could be produced synthetically by producing a composition with duplicate components.

Example 1 below describes a study to demonstrate the effect of diazepam (positive control) to inhibit spike-and-wave discharges induced by gamma-butyrolactone (GBL) administration in dogs (absence seizure model).

### REFERENCE EXAMPLE 1: EVALUATION OF DIAZEPAM FOR ANTICONVULSANT ACTIVITY IN DOG USING THE GBL INDUCED ABSENCE SEIZURE MODEL

The effect of diazepam as a positive control was tested in dogs using a pharmacologically induced model, the GBL induced absence seizure model. This model is a good predictor of treatment of seizures generally in canines. Diazepam is a known anticonvulsant and is commonly used as a positive control in seizure studies. However, diazepam is not suitable for maintenance treatment of epilepsy in dogs as the duration of its effect is too short.

Absence seizures are generalized non-convulsive seizures characterized by a brief loss of consciousness and spike-and-wave discharges in an electroencephalogram (EEG).¹¹

Gamma-hydroxybutyric acid (GHB) produces a pattern of EEG and behavioural changes when given to animals, which represent an experimental model of generalized absence seizures. GBL, a prodrug of GHB, similarly produce such changes when given to animals and it was found to produce these changes more rapidly and consistently than GHB, such that animals treated with GBL is a more reproducible and predictable model of absence seizures. ¹²

### Methods

### Study Details:

The experiment was carried out on nine male non-naïve beagle dogs, weighing 9 - 15 kg. All animals were housed in a box or in a kennel under natural lighting with restricted access to food (approx. 300 g/animal/day) and unlimited access to water.

### Surgery

The day prior to surgery, a trans-dermic patch of fentanyl as analgesic was applied to the animals. They also received amoxicillin as an antibiotic.

The day of surgery, the animals were premedicated with acepromazine used as tranquilizer and atropine sulfate to prevent bradycardia during anaesthesia and tracheal intubation. A few minutes later, the animals were anaesthetized with propofol. They were then placed under isoflurane anesthesia up to the end of the surgery and artificially ventilated.

Surgery was carried out under aseptic conditions.

Dogs were implanted with an Emka Technologies telemetric device. Following an incision in the lateral neck region, the telemetric device was introduced into a pouch under the skin. The positive and negative biopotential electrodes were coiled into a loop and tunnelled subcutaneously to protrude into the opened scalp incision. The skin incisions then were closed.

Following a sagittal incision of the scalp, the top of the skull was prepared for implantation. The incision was performed between bregma and lambda sutures in order to place electrodes over the parietal cortex. Three small holes were drilled into the skull, on the right hemisphere. The tips of the electrodes were then pushed through the holes through the skull onto the dura mater. The electrodes were connected to small plugs, placed into a connector and the whole assembly was secured on skull with dental cement. The skin incisions then were closed using aseptic chirurgical thread.

At the end of the day of surgery, the animals received Carprofen for post-operative pain and inflammation.

The dogs were housed individually in a box, during the first days after surgery.

Amoxicillin was given 1 day and 3 days after surgery for post-operative antibiotic management.

Carprofen was given once daily for 2 days after surgery for post-operative pain and inflammation management.

Approximately 2 weeks later, the dogs were placed in a sling for the experiment.

### EEG trace monitoring

For EEG recording, a telemetry receiver was located nearby the animal to record EEG.

All generated data was acquired and analysed using the EMKA Technologies software. The quality of the EEG signals was checked before launching EEG recording.

Before the experiment, EEG signals were recorded during a few minutes in baseline conditions to check the quality of the signals.

The baseline EEG recording was done for each dog, one day before the test session and lasted 40 minutes. Baseline and test sessions were done at the same time of the day for each dog.

### Test Compounds:

Test substance: Diazepam, white powder, dispersed in 0.2% hydroxypropylmethylcellulose (HPMC) in distilled water.

Vehicle control: 0.2% HPMC in distilled water.

Reagent: Gamma butyrolactone (GBL), colourless liquid, dissolved in physiological saline.

### Treatment schedule:

Each animal received 2 treatments, one tested per week, of vehicle or diazepam.

Diazepam (2 mg/kg) was administered orally 60 minutes before GBL injection and was compared with the corresponding vehicle. Then, each dog received GBL (500 mg/kg) by a single intravenous injection (bolus administration over 3 minutes, 1 mL/kg) 60 minutes after Diazepam administration.

EEG signals were recorded during 40 minutes to evaluate spike-and-wave discharges. The latency to observe spike-and-wave discharges was determined off-line on EEG signals.

### Data recording and analysis

The differential outputs between the 2 electrodes at each implantation site were digitized on-line at a sampling rate of 500 Hz/channel and the data stored in raw data files.

EEG was quantitatively analysed by spectral analysis using a Fast Fourier Transform algorithm. Spectra were calculated from epochs of 4.1-second duration. The mean total spectral power between 1.5 and 64 Hz and the power in 6 sub-frequency bands per brain structure recorded (1.5-4 (delta), 5-8 (theta), 8.5-12.5 (alpha), 13-35 (beta), 36-64 (gamma) and 3-6 Hz) was calculated on four 10-minute periods (from 0 to 10, 10 to 20, 20 to 30 and 30 to 40 minutes during baseline and test sessions).

Before being submitted to further analysis, all spectra containing recording artefacts found on visual inspection of the EEG traces were eliminated. Then the averages of 10-minute intervals of the verified individual spectra were saved into data files which then were transferred into Excel calculation sheets.

In order to account for inter-individual variability in the EEG amplitudes, results were expressed as percentage change in the absolute power of corresponding spectra between vehicle and test substance.

Data was analysed by comparing absolute values for each time-interval and each frequency band the tested group with the vehicle group. Statistical analysis was carried out using paired student's t test.

### Results

Motor incoordination and psychotic effects (stereotypies, involuntary movements, barks) were observed in three dogs administered with diazepam at 4 mg/kg during the test, i.e. after GBL administration.

At 6 mg/kg of diazepam, the symptoms were stronger. The dogs were afraid after administration of diazepam and were difficult to handle. It was thus not possible to administer GBL in these two dogs.

After these exclusions, 4 dogs were included in the analysis.

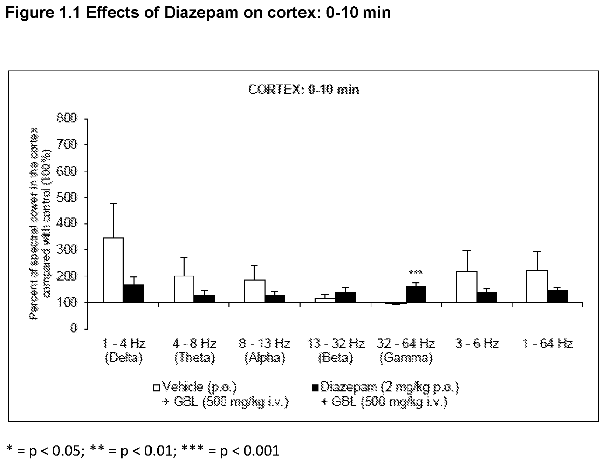

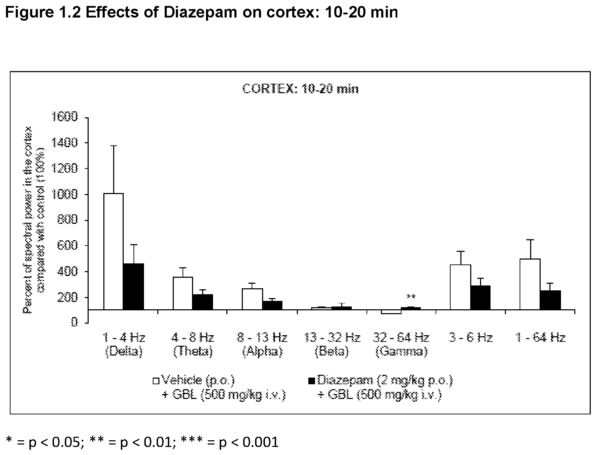

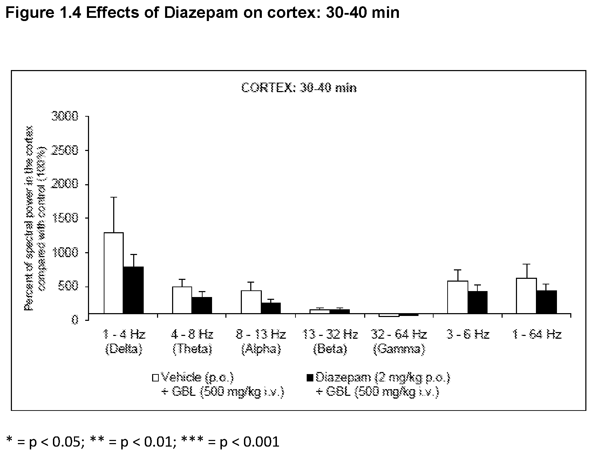

Figures 1.1 to 1.5 show the data produced in this experiment.

Diazepam is a member of the benzodiazepine family and benzodiazepine-induced EEG changes have been described in literature. The general consensus is that these changes include an increase in beta and a reduction in alpha band spectral power,¹³ and are associated with reductions in seizure burden. Thus, decreases in the lower frequency bands (delta, theta and alpha) and increases in the higher frequency bands (beta and gamma) were expected with diazepam.

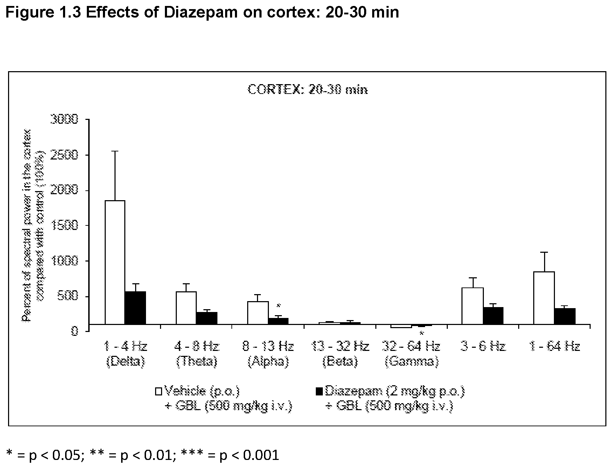

As shown in Figure 1.3, Diazepam (2 mg/kg) administered p.o. (per os) 60 minutes before GBL significantly decreased spectral power on alpha frequency band on the time-interval 20-30 minutes (p < 0.05), as compared with vehicle controls.

Spectral power on the gamma frequency band was significantly increased on the time-intervals 0-10 minutes, 10-20 minutes and 20-30 minutes (p < 0.001, p < 0.01 and p < 0.05, respectively), as shown in Figure 1.1 to 1.3.

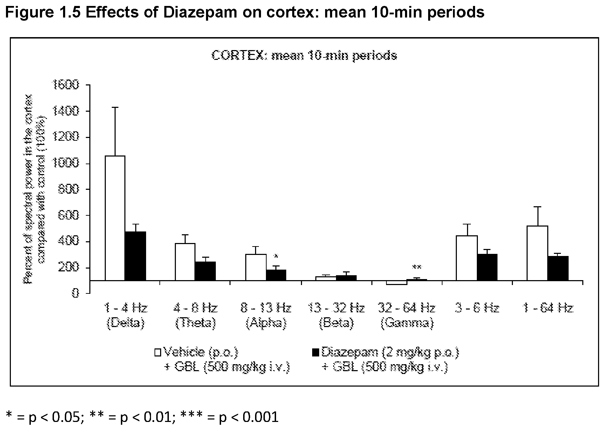

Similar effects of decreases in lower frequency bands and increases in high frequency bands were observed in the mean 10-minute periods (see Figure 1.5).

Overall, across all four time periods, the spectral power of lower frequency bands (delta, theta and alpha) decreased when diazepam was administered whilst the power of high frequency bands (beta and gamma) increased.

### Conclusions

These results demonstrate that Diazepam at 2 mg/kg p.o. lowered spectral power on 3-6 Hz frequency band following GBL administration in the dog as compared to vehicle control.

These data demonstrate the anticonvulsant effect of diazepam in this GHB model, affirming its role as a positive control.

Example 2 below describes the use of a botanically derived purified CBDV in a study to investigate the efficacy of purified pharmaceutical cannabidivarin formulation (CBDV) in the treatment of canine epilepsy. The study was carried out using the GBL model of example 1.

### EXAMPLE 2: EVALUATION OF CANNABIVIDARIN FOR ANTICONVULSANT ACTIVITY IN DOG USING THE GBL INDUCED ABSENCE SEIZURE MODEL

The efficacy of CBDV was tested in dogs using the GBL model of Example 1.

### Methods

### Study Details:

The experiment was carried out on three male non-naïve beagle dogs, weighing 9 - 15 kg. All animals were housed in a box or in a kennel under natural lighting with restricted access to food (approx. 300 g/animal/day) and unlimited access to water.

### Surgery

Surgery was carried out as detailed in Example 1.

### EEG trace monitoring

EEG recording was carried out as detailed in Example 1.

### Test Compounds:

Test substance: CBDV (50 mg/ml) in Labrafil^{®}
Vehicle control: Labrafil^{®} (1 mL/kg).
Reagent: Gamma butyrolactone (GBL), colourless liquid, dissolved in physiological saline.

### Treatment schedule:

The day of the test, the dogs were weighed prior to dosing.
Each animal received 2 treatments, one tested per week, of vehicle or CBDV.
CBDV (100 mg/kg) or vehicle was administered orally by gavage, 4 hours before GBL administration. Each dog received GBL (500 mg/kg) by a single intravenous injection (bolus administration over 3 minutes, 1 mL/kg) 4 hours after CBDV administration.

EEG signals were recorded during 40 minutes to evaluate spike-and-wave discharges. The latency to observe spike-and-wave discharges was determined off-line on EEG signals.

### Blood sampling

Blood samples of 2 mL was collected by puncture of the jugular or cephalic vein in all animals at the end of the experiment (around 4 hours and 40 minutes after CBDV administration).

Blood was collected using an identified vacuum tube containing Lithium Heparin tubes for plasma preparation. After sealing the tube, the blood sample was gently agitated and stored on ice until centrifugation (within 30 minutes of sampling). The blood samples were centrifuged at +4°C, at 2000g, for 10 minutes. The entire resultant plasma were immediately transferred into suitably labelled polypropylene tubes. The tubes were stored upright at approximately -80°C until transport to the Test Site for bioanalysis.

The samples were appropriately identified with at least the study number, test formulation, dose level, test session, animal identification, sampling date and sampling time.

### Data recording and analysis

Data was recorded and analysed as detailed in Example 1.

### Results

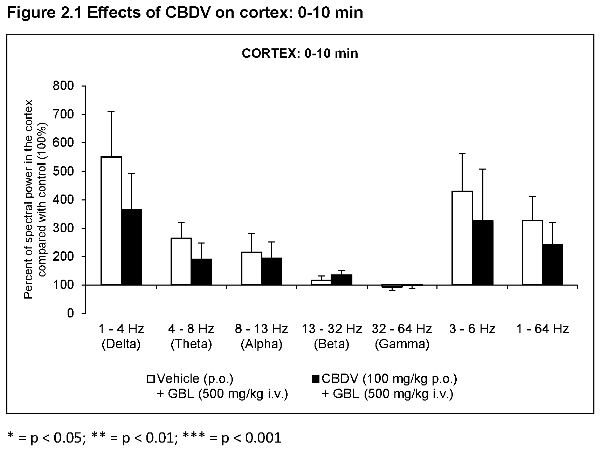

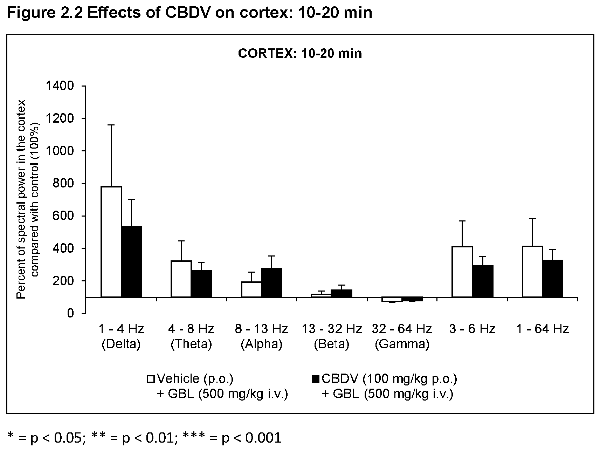

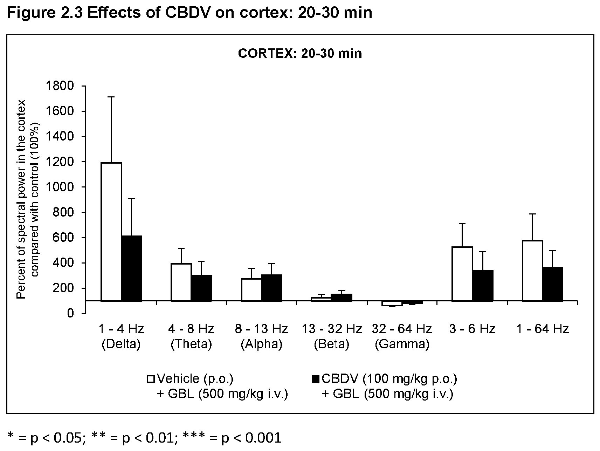

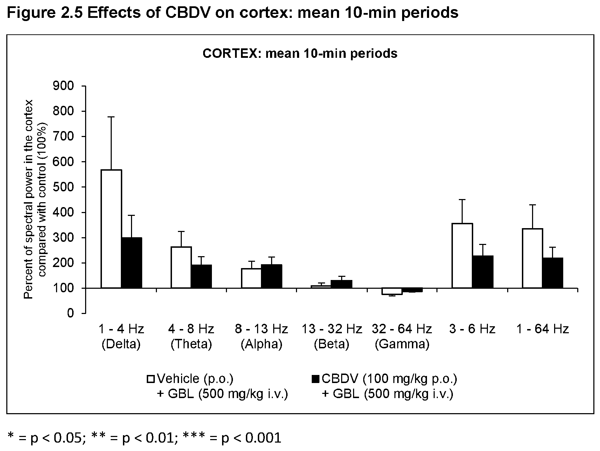

Figures 2.1 to 2.5 show the data produced in this experiment.

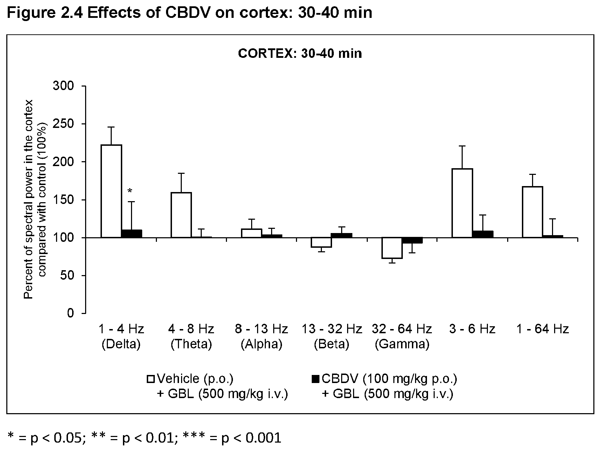

As shown by Figure 2.4, CBDV administered p.o. 4 hours before GBL significantly decreased spectral power on delta frequency bands at the time-interval 30-40 minutes (p < 0.05), as compared with vehicle controls.

Overall, across all four time periods, the spectral power of lower frequency bands (delta, theta and alpha) decreased when CBDV was administered whilst the power of high frequency bands (beta and gamma) increased, as was similarly found for diazepam in Example 1.

### Conclusions

These results demonstrate that CBDV at 100 mg/kg p.o. lowered spectral power on 3-6 Hz frequency band following GBL administration in the dog as compared to vehicle control.

These data are significant as they provide heretofore unknown evidence that this cannabinoid derivative CBDV may be of therapeutic value to treat canine epilepsy.

Clearly the compound produced a similar effect as diazepam in Example 1, suggesting that this compound exhibits anticonvulsive properties. Unlike diazepam, however, no adverse events were reported for CBDV administered at 100 mg/kg. After treatment with CBDV significant decreases in spectral power were observed at 30-40 minute period in the delta frequency band, when compared to vehicle.

### References

1. Heske et al. (2014) "A cohort study of epilepsy among 665,000 insured dogs: incidence, mortality and survival." Vet J; 202:471-6.
2. Podell et al. (2015) "ACVIM small animal consensus statement on seizure management in dogs." J Vet Intern Med 2016;30:477-490.
3. Volk et al. (2008) "The efficacy and tolerability of levetiracetam in pharmacoresistant epileptic dogs." Vet J 2008;176:310-319
4. Volk et al. (2015) "International Veterinary Epilepsy Task Force consensus report on epilepsy definition, classification and terminology in companion animals." BMC Vet Res 11:182.
5. McGrath et al. (2019) "Randomized blinded controlled clinical trial to assess the effect of oral cannabidiol administration in addition to conventional antiepileptic treatment on seizure frequency in dogs with intractable idiopathic epilepsy." Journal of the American Veterinary Medical Association. June 1, 2019, Vol. 254, No. 11, Pages 1301-1308
6. Hill et al. (2013) "Cannabidivarin-rich cannabis extracts are anticonvulsant in mouse and rat via a CB1 receptor-independent mechanism." Br J Pharmacol 2013 Oct;170(3):679-92. doi: 10.1111/bph.12321.
7. Hill et al. (2012) "Cannabidivarin is anticonvulsant in mouse and rat." British Journal of Pharmacology, Vol 167, pp 1629-1642. DOI:10.1111/j.1476-5381.
8. Berk et al. (2018) "Investigating owner use of dietary supplements in dogs with idiopathic epilepsy", pp 276-284. Research in Veterinary Science, Vol 119
9. Huizenga et al. (2019) "Preclinical safety and efficacy of cannabidivarin for early life seizures." Neuropharmacology 2019 Apr;148:189-198. doi: 10.1016/j.neuropharm.2019.01.002
10. "GW Pharmaceuticals Announces Preliminary Results of Phase 2a Study for its Pipeline Compound GWP42006" https://www.globenewswire.com/news-release/2018/02/21/1372900/0/en/GW-Pharmaceuticals-Announces-Preliminary-Results-of-Phase-2a-Study-for-its-Pipeline-Compound-GWP42006.html Accessed 5 Feb 2021
11. Lee et al. (2019) "Distinct Topographical Patterns of Spike-Wave Discharge in Transgenic and Pharmacologically Induced Absence Seizure Models." Exp Neurobiol. 2019 Aug; 28(4): 474-484.
12. Snead et al. (1991) "The gamma-hydroxybutyrate model of absence seizures: correlation of regional brain levels of gamma-hydroxybutyric acid and gamma-butyrolactone with spike wave discharges." Neuropharmacology 1991 Feb;30(2):161-7. doi: 10.1016/0028-3908(91)90199-I.
13. Wilson et al. (2014). "Can pharmaco-electroencephalography help improve survival of central nervous system drugs in early clinical development?" Drug Discovery Today; Volume 19, Number 3; March 2014

## Claims

1. A cannabidivarin (CBDV) preparation for use in the treatment of seizures associated with canine epilepsy, wherein the dose of CBDV is between 75 mg/kg/day and 125 mg/kg/day, wherein the CBDV preparation comprises greater than 95% (w/w) CBDV and not more than 1.5% (w/w) tetrahydrocannabinol (THC).

2. A CBDV preparation for use according to claim 1, wherein the seizures associated with epilepsy are tonic, clonic, tonic-clonic, atonic, myoclonic, absence, focal seizures without impairment, focal seizures with impairment, and focal seizures with secondary generalisation.

3. A CBDV preparation for use according to any of the preceding claims, wherein the CBDV preparation comprises greater than or equal to 95% (w/w) CBDV and less than or equal to 5% (w/w) other cannabinoids, wherein the less than or equal to 5% (w/w) other cannabinoids comprise the cannabinoids tetrahydrocannabinol (THC); tetrahydrocannabivarin (THCV); cannabidiol-C1 (CBD-C1); cannabidiol (CBD); cannabidivarin acid (CBDVA) and cannabidiol-C4 (CBD-C4).

4. A CBDV preparation for use according to any of the preceding claims, wherein the CBDV preparation is used in combination with one or more concomitant anti-epileptic drugs (AED).

5. A CBDV preparation for use according to any of the preceding claims, wherein the CBDV is present is isolated from cannabis plant material.

6. A CBDV preparation for use according to any of the preceding claims, wherein at least a portion of at least one of the cannabinoids present in the CBDV preparation is isolated from cannabis plant material.

7. A CBDV preparation for use according to claims 1 to 4, wherein the CBDV is present as a synthetic preparation.

8. A CBDV preparation for use according to claim 7, wherein at least a portion of at least one of the cannabinoids present in the CBDV preparation is prepared synthetically.

9. A CBDV preparation for use according to any of claims 1 to 8, wherein the dose of CBDV is 100 mg/kg/day.

10. A CBDV preparation for use according to claim 9, wherein the preparation is administered orally.

## Patentansprüche

1. Cannabidivarin(CBDV)-Präparat zur Verwendung bei der Behandlung von Anfällen im Zusammenhang mit Epilepsie bei Hunden, wobei die Dosis von CBDV zwischen 75 mg/kg/Tag und 125 mg/kg/Tag liegt, wobei das CBDV-Präparat mehr als 95 Gew.-% CBDV und nicht mehr als 1,5 Gew.-% Tetrahydrocannabinol (THC) umfasst.

2. CBDV-Präparat zur Verwendung nach Anspruch 1, wobei die Anfälle im Zusammenhang mit Epilepsie tonische, klonische, tonisch-klonische, atonische, myoklonische, Absence-, fokale Anfälle ohne Beeinträchtigung, fokale Anfälle mit Beeinträchtigung und fokale Anfälle mit sekundärer Generalisierung sind.

3. CBDV-Präparat zur Verwendung nach einem der vorhergehenden Ansprüche, wobei das CBDV-Präparat mehr als oder gleich 95 Gew.-% CBDV und weniger als oder gleich 5 Gew.- % andere Cannabinoide umfasst, wobei die weniger als oder gleich 5 Gew.-% anderen Cannabinoide die Cannabinoide Tetrahydrocannabinol (THC); Tetrahydrocannabivarin (THCV); Cannabidiol-C1 (CBD-C1); Cannabidiol (CBD); Cannabidivarinsäure (CBDVA) und Cannabidiol-C4 (CBD-C4) umfassen.

4. CBDV-Präparat zur Verwendung nach einem der vorhergehenden Ansprüche, wobei das CBDV-Präparat in Kombination mit einem oder mehreren begleitenden Antiepileptika (AED) verwendet wird.

5. CBDV-Präparat zur Verwendung nach einem der vorhergehenden Ansprüche, wobei das vorliegende CBDV aus Cannabispflanzenmaterial isoliert ist.

6. CBDV-Präparat zur Verwendung nach einem der vorhergehenden Ansprüche, wobei mindestens ein Teil mindestens eines der in dem CBDV-Präparat vorliegenden Cannabinoide aus Cannabispflanzenmaterial isoliert ist.

7. CBDV-Präparat zur Verwendung nach den Ansprüchen 1 bis 4, wobei das CBDV als ein synthetisches Präparat vorliegt.

8. CBDV-Präparat zur Verwendung nach Anspruch 7, wobei mindestens ein Teil mindestens eines der Cannabinoide, die in dem CBDV-Präparat vorliegen, synthetisch zubereitet ist.

9. CBDV- Präparat zur Verwendung nach einem der Ansprüche 1 bis 8, wobei die Dosis von CBDV 100 mg/kg/Tag beträgt.

10. CBDV-Präparat zur Verwendung nach Anspruch 9, wobei das Präparat oral verabreicht wird.

## Revendications

1. Préparation de cannabidivarine (CBDV) destinée à être utilisée dans le traitement de crises associées à l'épilepsie canine, dans laquelle la dose de CBDV est comprise entre 75 mg/kg/jour et 125 mg/kg/jour, dans laquelle la préparation de CBDV comprend plus de 95 % (p/p) de CBDV et pas plus de 1,5 % (p/p) de tétrahydrocannabinol (THC).

2. Préparation de CBDV destinée à être utilisée selon la revendication 1, dans laquelle les crises associées à l'épilepsie sont toniques, cloniques, tonico-cloniques, atoniques, myocloniques, d'absence, des crises focales sans altération, des crises focales avec altération et des crises focales avec généralisation secondaire.

3. Préparation de CBDV destinée à être utilisée selon l'une quelconque des revendications précédentes, dans laquelle la préparation de CBDV comprend au moins 95 % (p/p) de CBDV et au plus 5 % (p/p) d'autres cannabinoïdes, dans laquelle les autres cannabinoïdes présents à au plus 5 % (p/p) comprennent les cannabinoïdes tétrahydrocannabinol (THC) ; tétrahydrocannabivarine (THCV) ; cannabidiol-C1 (CBD-C1) ; cannabidiol (CBD) ; acide cannabidivarine (CBDVA) et cannabidiol-C4 (CBD-C4).

4. Préparation de CBDV destinée à être utilisée selon l'une quelconque des revendications précédentes, dans laquelle la préparation de CBDV est utilisée en combinaison avec un ou plusieurs médicaments anti-épileptiques (AED) concomitants.

5. Préparation de CBDV destinée à être utilisée selon l'une quelconque des revendications précédentes, dans laquelle la CBDV présente est isolée à partir de matière végétale de cannabis.

6. Préparation de CBDV destinée à être utilisée selon l'une quelconque des revendications précédentes, dans laquelle au moins une partie d'au moins l'un des cannabinoïdes présents dans la préparation de CBDV est isolée à partir de matière végétale de cannabis.

7. Préparation de CBDV destinée à être utilisée selon les revendications 1 à 4, dans laquelle la CBDV est présente sous forme d'une préparation synthétique.

8. Préparation de CBDV destinée à être utilisée selon la revendication 7, dans laquelle au moins une partie d'au moins l'un des cannabinoïdes présents dans la préparation de CBDV est préparée de manière synthétique.

9. Préparation de CBDV destinée à être utilisée selon l'une quelconque des revendications 1 à 8, dans laquelle la dose de CBDV est de 100 mg/kg/jour.

10. Préparation de CBDV destinée à être utilisée selon la revendication 9, dans laquelle la préparation est administrée par voie orale.
